# EUROPEAN PATENT APPLICATION

(11) **EP 1 813 934 A1**
(43) Date of publication of application: **01.08.2007**
(21) Application number: 05805472.7
(22) Date of filing: 01.11.2005
(51) Int. Cl.: G01N 21/88, G01B 11/30, G01N 29/04, G06F 17/50

(54) **CONCRETE STRUCTURE CRACK INSPECTION DEVICE AND CRACK INSPECTION METHOD**

(30) Priority: 02.11.2004 JP 2004319415
(71) Applicant: Hara, Tooru, Nada-ku Kobe City, Hyogo 657-0804 (JP); Kobori, Kenichi, Nara 630-8101 (JP)
(72) Inventor: KOBORI, Kenichi, 6308101 (JP)
(74) Representative: Sunderland, James Harry
(86) International application number: PCT/JP2005/020118
(87) International publication number: WO 2006/049174

(57) **Abstract**

A crack inspection device (6) for a concrete structure (1) includes: storage means for storing raster data obtained by imaging a surface (2) of the concrete structure (1) having a crack (3); conversion means for performing raster vector conversion on the data obtained by digitizing the raster data; data superimposing means for superimposing the vector data of the crack (3) obtained by the conversion means on the graphic data of the surface (2) of the concrete structure (1) while matching the coordinates; and output means for outputting the superimposed data obtained by the data superimposing means.

## Description

### Technical field

The present invention relates to a crack inspection device and a crack inspection method of a concrete structure, capable of grasping correctly the actual condition of cracks generated on the surface of the concrete structure, and the proof stress of the concrete structure without destroying it.

### Background Art

When the state of cracks generated on the surface of a comparatively large-sized concrete structure which has a height of several meters, for example, the concrete structure such as a revetment built on a slope of a mountain, a dam and the like, is inspected, usually, a worker checks the cracks with eyes and makes sketches in handwriting.
However, in the crack inspection method depending on such sketches, there are no criteria for checking the sketches to the actual crack, and the length and the pattern of the cracks is influenced by the subjectivity of the worker who sketches them, and further thin cracks may be overlooked, or the cracks having a complicated pattern may not be drawn, so that it is very inaccurate.

Therefore, as a method for inspecting the cracks of the above concrete structure, an inspection method is developed recently, in which an image of the cracks generated in the surface of the concrete structure is taken by a digital image pick-up machine, such as a digital camera and a CCD camera, and image processing of raster data of the digital image obtained thereby is carried out so as to grasp the width, the area, etc. of the cracks.

And in the conventional crack inspection method, after the image is taken and then the raster data is binarized, the number of pixels judged as cracks is counted so as to obtain the area and the width of the cracks, and the sum total of the brightness values of the respective pixels is regarded as the appearance area of the cracks, and the area of the cracks is obtained from this appearance area (Japanese patent document, Japanese Laid Open Patent No. 2003-214827).

However, since in the conventional inspection method of the cracks, the image processing is performed by using the raster data obtained with the digital image pick-up machine, without any change, the cracks can be grasped with markedly sufficient accuracy when counting the number of pixels or totaling the brightness values of the respective pixels, in comparing with the sketches by a manpower, and since the amount of data for expressing one crack, however, becomes huge, and information of the position, shape and the like lacks in the raster data itself, there are drawbacks that the internal structure of the concrete structure which has cracks cannot be expressed in three dimension, the concrete structure cannot be used for the strength calculation in an FEM analysis, or neither crack length nor crack volume can be calculated automatically.

### Disclosure of the Invention

It is an object of the present invention to allow three dimension representation and its strength analysis of the internal structure of the concrete structure which has cracks, to be performed by expressing correctly the positions and shapes of cracks which have been generated on the surface of the concrete structure, with the smaller amount of data.

In order to achieve the object, the crack inspection device for a concrete structure according to the present invention, comprises a memory unit which stores raster data obtained by taking an image of a surface of the concrete structure having a crack, a conversion unit which performs a raster vector conversion process to data obtained by binarizing the raster data, a data synthesizing unit which matches and synthesizes the vector data of the crack obtained by the conversion unit and figure data of the surface of the concrete structure, according to coordinates, and an output unit which outputs the synthesized data obtained by the data synthesizing unit.

Since, in the crack inspection device of the present invention, after binarizing the raster data of the cracks stored in the memory unit, a raster vector conversion processing is carried out thereto, the vector data of the cracks obtained thereby is synthesized and matched with figure data of the surface of the concrete structure, according to coordinates, the positions and shapes of cracks which have been generated in the surface of the concrete structure can be outputted correctly in form of vector data whose amount of data is much smaller than that of raster data.

Moreover, since the crack vector data itself has information of the positions, shapes and the like, while the vector data is added to figure data of the surface of the concrete structure in order to make drawings, the numeric data of the crack depths corresponding to the vector data of the cracks is added thereto, so as to generate three dimension data, as described below, so that the internal structure of the concrete structure which has cracks can be expressed in three dimension, or strength calculation of the concrete structure can be carried out by an FEM analysis.

Since image data of reference points marked on the surface of the concrete structure beforehand is contained in the raster data, the crack inspection device of the present invention needs to have a compensation unit to perform distortion compensation of the raster data based on the image data of the reference points.

In this case, since the distortion compensation of the raster data of the cracks is performed based on the image data of the reference points, even in case where an image is taken at a slanted angle with respect to the surface of the concrete structure, or even in case of raster data which curves toward an end portion from the central portion, exact plane shape raster data can be obtained by the compensation.

On the other hand, although the number of pixels of a digital image pick-up machine, such as a digital camera used with the crack inspection device of the present invention, is more preferably large, if, for the reasons of accuracy, the entire surface of the concrete structure cannot be covered by one time shot imaging, the raster data may comprise two or more ones which are obtained by taking images of parts of the surface of the concrete structure, and in this case, what is necessary is just to cover the entire surface with two or more of pieces of raster data. But it is necessary to have a joining unit to join the obtained pieces of vector data of the crack, which correspond to the respective parts of raster data.

Since, in the crack inspection device of the present invention, the vector data of the cracks obtained by carrying out a raster vector conversion process after binarizing the raster data obtained with the digital image pick-up machine, in itself is two dimension data, at least the crack depth is needed to use it for image rendering or an FEM analysis of the internal structure of the concrete structure. Then, in order to perform the three dimension-image processing or the strength calculation, a crack inspection method as set forth below is recommended.

Namely, the crack inspection method of the present invention, includes, a step of obtaining the vector data of the cracks by carrying out a raster vector conversion process after binarizing the raster data obtained by taking an image of the surface of the concrete structure which has cracks, a step of measuring the crack depths of the cracks by ultrasonic detection, a step of adding the numeric data of the crack depths to the vector data of the cracks corresponding to the positions where the detection is performed, so as to generate three dimension data, a step of synthesizing and matching the three dimension data of the cracks and three dimension figure data of the concrete structure, and a step of outputting the synthesized three dimension data obtained at the step.

Moreover, based on the synthesized three dimension data of the concrete structure, an FEM analysis of an arbitrary cross section of the structure can also be performed.

In this case, core boring etc. is not necessary and measurement cost becomes low, since the crack depths of a cracks are measured by ultrasonic detection, and since the numeric data of the crack depths is added to the vector data of the cracks corresponding to the positions where the detection is carried out so as to generate three dimension data, the three dimension data can be quantified more in accuracy, compared with the case where the numeric data of the crack depths is added to the cracks by worker's sketches.

In addition, as the ultrasonic detection, a nondestructive inspection method disclosed in Japanese patent document, Japanese Laid Open Patent No. 2004-184276 can be adopted.

According to the present invention, since the positions and shapes of cracks which have been generated in the surface of the concrete structure can be correctly expressed with the smaller amount of data, three dimension representation of the internal structure of the concrete structure which has cracks, and its strength analysis can be performed.

### Brief Description of the Drawings

[Fig. 1] It is a perspective diagram showing an example of a crack inspection device according to the present invention.
[Fig. 2] It is a flowchart of an image processing performed by the inspection device of Fig. 1.
[Fig. 3] It is a flowchart of image processing and an FEM analysis etc. performed by the inspection device of Fig. 1.
[Fig. 4] It is a crack distribution map (front view) of a dam which has cracks, shown by CAD data.
[Fig. 5] It is a front view of a display in which a display example of vector data (two dimension data) of cracks is shown.
[Fig. 6] It is a front view of a display in which a display example of vector data (three dimension data) of cracks to which crack depths are added, is shown.

### The Best Form for Carrying out the Invention

Hereafter, embodiments of the present invention will be explained, based on drawings.

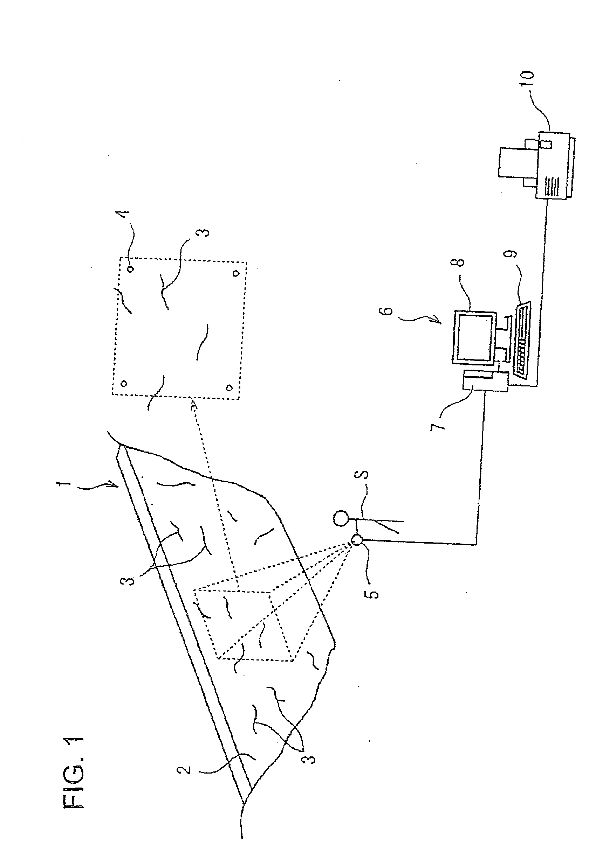

Fig. 1 is a schematic view of an example of a crack inspection device of the concrete structure according to the present invention. As shown in this figure, in this embodiment, a dam is selected as a concrete structure 1 which is the subject of examination of cracks, and many cracks 3 have occurred in a surface 2 of this dam 1. This dam 1 has a comparatively large-sized concrete structure in which upside cracks 3 cannot be reached, unless a scaffold is built, and the width thereof is about twenty meters, and the maximum height of the central portion is about 10 meters.

Reference points 4 are beforehand marked on four corners of an exact rectangle area whose image should be taken, by a laser pointer (not shown) on the surface 2 of the dam 1. As shown as a broken line area in Fig. 1, a worker S who takes an image at the spot determines an image pick-up range so that these reference points 4 will come to the respective four corners, and an image of part of the surface of the dam 1 is taken with a digital image pick-up machine 5 which comprises a digital camera or a CCD camera. In addition, the digital image pick-up machine 5 whose number of pixels is eight million or more is desirable, in order to take an image of the cracks 3 as accurate as possible.

The image data (raster data: bmp, jpg, etc.) taken with the digital image pick-up machine 5 is transmitted to a personal computer 6 by wired or wireless connections, and is recorded in the computer 6. This computer 6 is equipped with a computer main body 7, a display 8 which is a CRT or liquid crystal display, a keyboard 9 and a mouse (not shown), and a color printer 10 is connected to the computer main body 7.

The computer main body 7 is equipped therein with a hard disk in which OS software, application software which performs various image processing mentioned later, and data, such as the image data, is stored, a main memory in which the software, image data, etc. is temporarily stored, and a control unit which comprises a CPU etc. and performs tasks based on commands from the software.

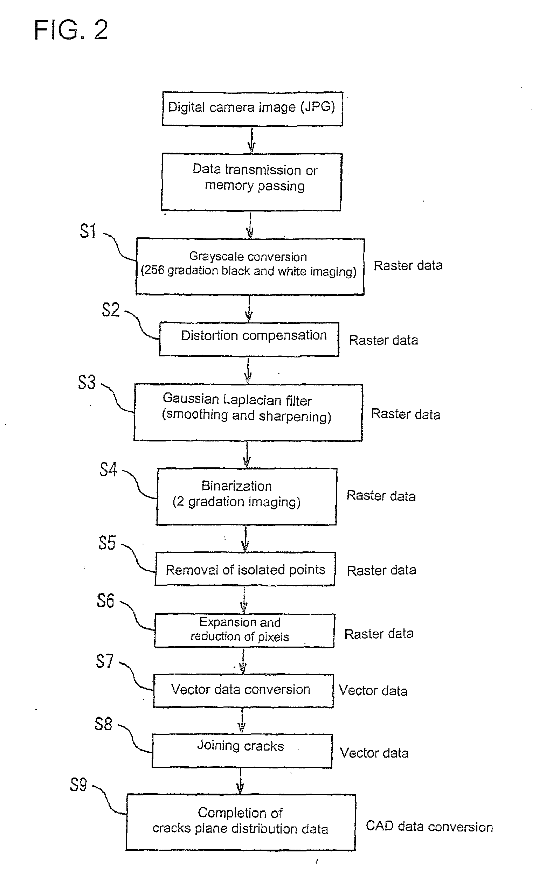

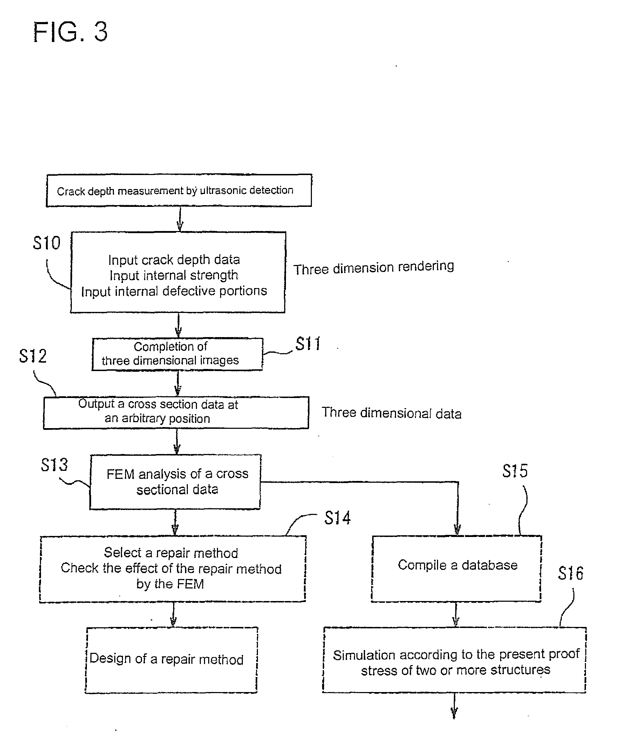

Hereafter, with reference to Figs. 2 and 3, the image processing performed in the computer 6 is explained in order.

As shown in Fig. 2, the raster data obtained by the digital image pick-up machine 5 is taken into the hard disk of the computer main body 7 by data transfer or memory passing, first, gray scale conversion is carried out (in this embodiment, black and white image of 256 gradation) (S1). Then, distortion compensation of the raster data is performed based on the image data of the reference points 4 (S2), so that even in case where an image is taken at a slanted angle with respect to the surface of the concrete structure 1, or even in case of raster data which curves toward an end portion from the central portion, exact plane shape raster data can be obtained by the compensation.

Next, the image is smoothed and sharpened by applying Gaussian Laplacian filter to the raster data whose distortion has been compensated as mentioned above (S3), only the image from which a noise is removed and corresponds to the cracks 3, stand out. After that, this image data is binarized based on a predetermined threshold (imaging of 2 gradation) (S4), the raster data is separated only into black and white, and then divided into cracks or non-cracks, and isolated-point removing processing is performed to the raster data of the cracks 3, which is binarized (S5). This isolated-point removing processing is one that removes points that are scattered and isolated in places which do not clearly relate to the cracks 3, in which the isolated points having a predetermined length or less are regarded as non-cracks so that they are automatically removed.

Then, expansion and reduction processing of pixels of the cracks 3 which are raster data are performed (S6). This processing is a task for determining break of continuity thereof, when the length of the pixel group of the raster data which forms the cracks 3 is short (when the continuity of the cracks 3 is broken), in which when the length thereof is less than a predetermined one, it is regarded as a broken line so as to be automatically divided into two or more cracks 3, and when the length thereof is equal to or more than the predetermined one, it is regarded as a crack 3, so that they are automatically connected, so as retain continuity thereof as one crack 3. After performing each processing to the image data containing the cracks 3, the raster vector conversion process is performed to the raster data which forms the respective cracks 3 (S7), in which each crack 3 is extracted as vector data, and processing for joining the vector data with vector data of other cracks 3 obtained from adjoining image data is performed (S8).

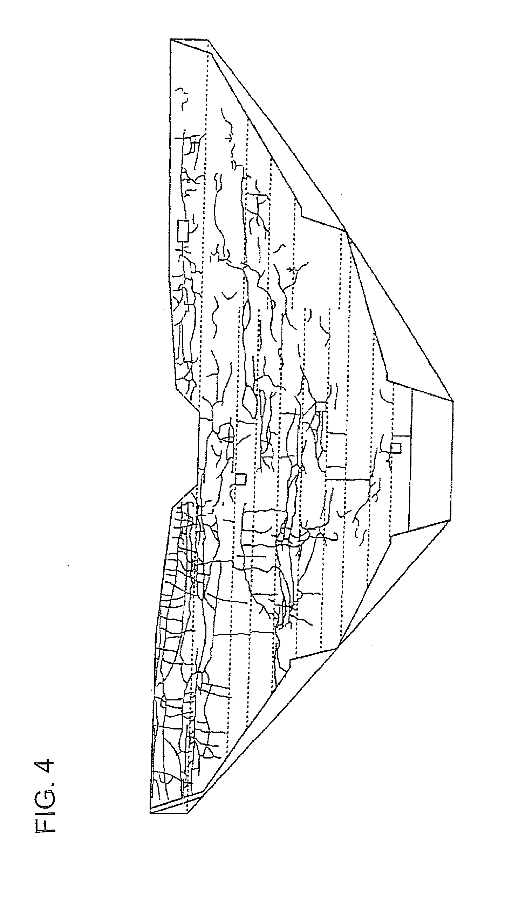

By matching and synthesizing, according to coordinates, the vector data of the respective cracks 3 obtained described above, to the figure data of the surface 2 of the dam 1 beforehand stored in the hard disk of the computer main body 7 (S9), for example, as shown in Fig. 4, a crack distribution map (CAD data) of the surface 2 of the dam 1 which has cracks 3 is obtained.

In addition, this crack distribution map can also be displayed on the display 8 of the computer 6, and can also be printed out by a printer 10. That is, in the present invention, the output unit of the crack distribution map which has been obtained by synthesizing the vector data of the cracks 3 and the figure data of the surface of the dam 1, may be either the display 8 or the printer 10.

Next, as shown in Fig. 3, based on the cracks distribution map, a worker S carries out crack depth measurement by ultrasonic detection with nondestructive inspection device (not shown) at the spot, the numeric data of the crack depths is added to the vector data of the cracks 3 corresponding to the positions where detection is carried out, thereby forming three dimension data (S10).

In this case, if the nondestructive inspection method disclosed in the Japanese Laid Open Patent No. 2004-184276 is adopted, since not only the crack depths but the internal strength and the internal defective portions of the dam 1 can be detected without destruction, the data is added to the CAD data which forms the crack distribution map, if needed (S10).

By matching and synthesizing, according to coordinates, the three dimension data of the cracks 3 and the three dimension figure data of the dam 1, the three dimension image which is the synthesized three dimension data of the dam 1 having the cracks 3 and to which the data of crack depth is added, is completed (S11). This three dimension image can be also displayed on the display 8, and can also be printed out by the printer 10.

Moreover, when the three dimension image is displayed on the display 8, it also possible to rotate the image with respect to an arbitrary rotational axis, so that the dam 1 having the cracks 3 can be observed from various directions.

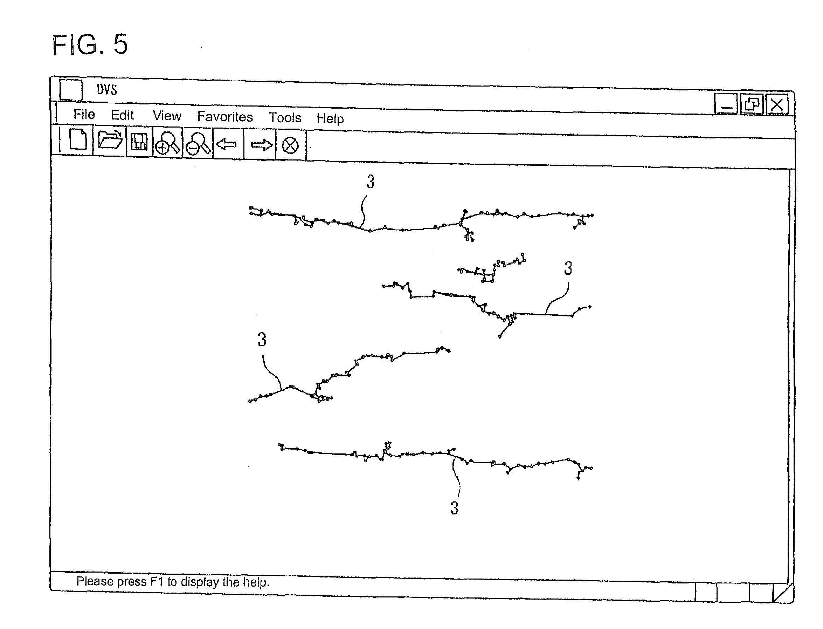

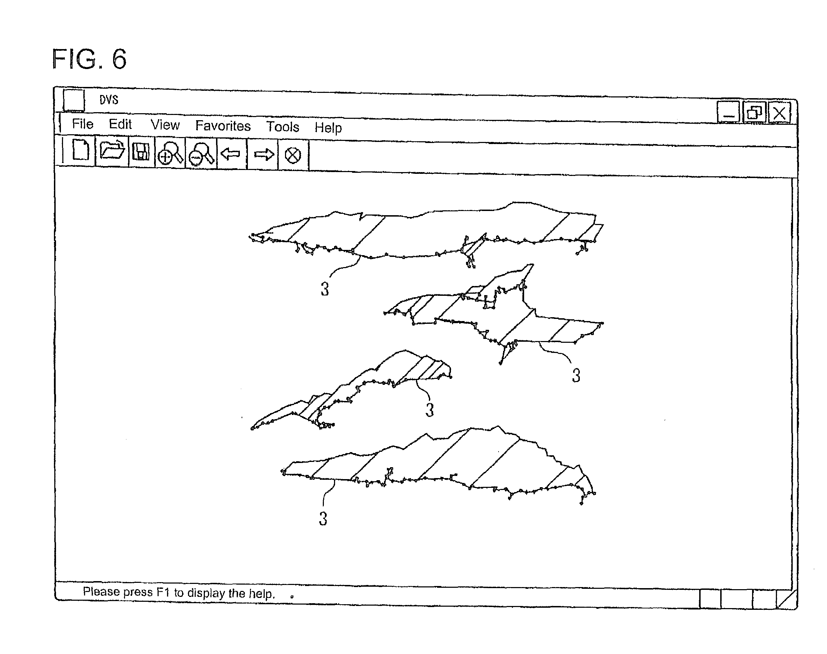

Fig. 5 shows an example at the time of displaying the vector data of the cracks 3 in two dimension on the display 8, and Fig. 6 shows an example at the time of displaying the vector data to which the crack depths are added, on the display 8. In the case of Fig. 6, the internal structure of the cracks 3 is displayed in three dimension by displaying the crack depths in the respective positions of the cracks 3 upper right in about 45 degree direction. In addition, the display method of the crack depths corresponding to the respective cracks 3 is not limited thereto, and may be shown by color coding or dark and light coloring.

Next, the image data in a cross section of an arbitrary width direction position is outputted using the three dimension image which is CAD data (S12), and an FEM analysis is performed based on the image data of the cross section, so that numerical calculation of the internal stress state of the dam 1 is carried out (S13).

Thus, when the internal stress state in the arbitrary cross section of the dam 1 in the FEM analysis is grasped, based on the result, a specific repair methods of the dam 1 (for example, injection of cement, placing of anchor bolts, etc. to a cracks) is selected (S14). Moreover, the effect of the repair method can be checked by inputting the state after the repair method is carried out, into the three dimension image of the dam 1, and performing the FEM analysis again (S14).

In addition, the total extending length of all the cracks 3 generated on the surface 2 of the dam 1 can be also automatically calculated from the two dimension vector data of the cracks 3, and based on this total extending length, the required quantity of a sealing agent to be applied to the surface 2 can also be grasped. Moreover, the total volume of all the cracks 3 generated on the surface of the dam 1 can be also automatically calculated from the three dimension vector data of the cracks 3, and based on the total volume, the required quantity of an impregnation agent to be impregnated into the cracks 3 can also be grasped.

If, in the same manner, the analysis result about not only the dam 1 but also other concrete structures in the neighborhood according to the FEM is put in a database (S15), it becomes possible to carry out a simulation about whether or not the proof stress thereof can endue external forces (increase in the earth pressure generated by a mudslide etc.) under the present condition of the two or more structures in the neighborhood including the dam 1 (S16).

In addition, the present invention is not limited to the above-mentioned embodiments.

For example, although the desktop computer is shown as the computer in Fig. 1, this computer 6 may be a portable computer.

Moreover, the image data or the measurement information of the ultrasonic detection acquired at the spot is once inputted into a portable computer, and the information is transmitted to a higher speed computer installed in a data centre through the Internet, so that in this computer, the image processing or the FEM analysis may be performed. In this case, it is sufficient if the worker S who can perform image pick-up work by the digital image pick-up machine 5 and measurement by ultrasonic detection apparatus is dispatched to the spot, and since it is not necessary to dispatch an IT expert skilled in the complicated data processing to the spot, a staff required for the crack inspection can be distributed efficiently.

## Claims

1. A crack inspection device for a concrete structure, comprising:
a memory unit which stores raster data obtained by taking an image of a surface of the concrete structure having a crack;
a conversion unit which performs a raster vector conversion process to data obtained by binarizing the raster data;
a data synthesizing unit which matches and synthesizes the vector data of the crack obtained by the conversion unit and figure data of the surface of the concrete structure, according to coordinates; and
an output unit which outputs the synthesized data obtained by the data synthesizing unit.

2. The crack inspection device for a concrete structure according to claim 1, further including a compensation unit, wherein the raster data includes image data of reference points which are attached beforehand to the concrete structure, and the compensation unit compensates distortion of the raster data based on the image data of the reference points.

3. The crack inspection device for a concrete structure according to claim 1 or 2, further including a joining unit, wherein the raster data comprises two or more piece of raster data obtained by taking images of parts of the surface of the concrete structure, so that the entire surface is covered by the two or more of raster data, and the joining unit joins the vector data of the cracks corresponding the respective obtained two or more pieces of raster data with each other.

4. A crack inspection method for a concrete structure comprising:
a step of storing raster data obtained by taking an image of a surface of the concrete structure having cracks;
a step of performing a raster vector conversion process to data obtained by binarizing the raster data;
a step of matching and synthesizing the vector data of the cracks and figure data of the surface of the above-mentioned concrete structure according to coordinates thereof; and
a step of outputting the synthesized data obtained in the step.

5. The crack inspection method for a concrete structure according to claim 4, further including a step of taking the image in a state where a reference point is attached beforehand to the surface of the concrete structure, and a step of compensating distortion of the raster data based on an image data corresponding to the reference point.

6. The crack inspection method for a concrete structure according to claim 4 or 5, further including a step of storing two or more pieces of raster data which covers the entire surface thereof by taking images of parts of the surface of the concrete structure, and joining the respective obtained vector data of the crack, which corresponds to the two or more pieces of raster data, with each other.

7. A crack inspection method for a concrete structure, comprising:
a step of obtaining vector data of a crack by carrying out a raster vector conversion process after binarizing raster data obtained by taking an image of the surface of the concrete structure having the crack;
a step of measuring a crack depth of the crack by ultrasonic detection;
a step of adding numeric data of the crack depth to the vector data of the crack corresponding to a position where the detection is performed, so as to generate three dimension data;
a step of matching and synthesizing the three dimension data of the crack and the three dimension figure data of the concrete structure according to coordinates; and
a step of outputting the synthesized three dimension data obtained in the step.

8. The crack inspection method for a concrete structure according to claim 7, wherein an FEM analysis of an arbitrary cross section of the structure is performed based on the synthesized three dimension data of the concrete structure.
